# FASCICULE DE BREVET EUROPEEN

(11) **EP 1 140 271 B1**
(45) Date de publication et mention de la délivrance du brevet: **23.07.2003**
(21) Numéro de dépôt: 99961150.2
(22) Date de dépôt: 22.12.1999
(51) Int. Cl.: A61M 25/02

(54) **DISPOSITIF DE MAINTIEN D'AU MOINS UN DRAIN**
VORRICHTUNG ZUR HALTERUNG FÜR MINDESTENS EINE MEDIZINISCHE DRAIN
DEVICE FOR MAINTAINING AT LEAST A TUBE

(30) Priorité: 22.12.1998 FR 9816247
(43) Date de publication de la demande: 10.10.2001
(73) Titulaire: Navarro, Francis, 34980 Monteferrier sur Lez (FR)
(72) Inventeur: Navarro, Francis, 34980 Monteferrier sur Lez (FR)
(74) Mandataire: Thinat, Michel
(86) Numéro de dépôt international: FR9903258
(87) Numéro de publication internationale: WO00037136

(56) Documents cités:
- EP-A- 0 082 596
- WO-A-91/07204
- WO-A-97/45148
- WO-A-98/25661
- GB-A- 2 128 481
- US-A- 5 569 207

## Description

L'invention concerne un dispositif de maintien d'au moins un drain ou analogue, passant par un orifice artificiel dans le corps d'un patient.

En chirurgie et en réanimation, il est souvent nécessaire d'effectuer un drainage thoracique ou abdominal. Le drainage consiste à mettre en communication l'intérieur du thorax ou de l'abdomen avec un système d'aspiration, en général pour évacuer un épanchement de la cavité pleurale ou abdominale ou encore drainer un site opératoire, ou un organe creux comme l'estomac, l'intestin, les voies biliaires, l'oesophage.

La fixation du drain est classiquement assurée par une suture à la peau, un pansement étant prévu autour du drain.

Ceci pose de nombreux problèmes.

La fixation par suture est douloureuse pour le patient du fait de la traction exercée sur le drain et c'est une source d'infection.

Le drain a tendance à se plier à proximité de l'orifice pratiqué dans le corps du patient, du fait de son raccordement au système d'aspiration. Ceci est également une source de douleurs pour le patient. De plus, la présence des pansements rend difficile l'accès au drain pour vérifier sa position ou encore la présence d'éventuelles sécrétions.

Enfin, quelques jours après l'intervention, le drain doit être retiré progressivement du corps du patient, en deux ou trois étapes.

A chaque fois, il faut couper le fil, éventuellement le fixer à la peau par une nouvelle suture. Il faut également refaire tous les pansements et notamment mettre en place une nouvelle poche de recueil de sécrétions.

Ces manipulations sont douloureuses pour le patient, constituent un risque d'infection au niveau de l'orifice cutané, un travail important pour le personnel soignant. Elles sont coûteuses tant en main d'oeuvre qu'en produits utilisés.

C'est pourquoi différents systèmes ont été mis au point pour maintenir de tels drains.

On peut notamment citer le document FR-2 707 175 qui décrit un dispositif réalisé en deux éléments séparables sensiblement semi-cylindriques et qui est fixé au corps du patient au moyen d'une zone adhésive. De plus, le pansement peut être remplacé par une pièce en un matériau absorbant, placée entre le dispositif et la zone adhésive. Le drain s'étend sensiblement perpendiculairement au corps du patient, à proximité du corps.

Ce dispositif permet d'assurer de façon efficace le maintien du drain sur le corps du patient, sans qu'une suture à la peau soit nécessaire.

Cependant, un tel dispositif ne permet pas de donner au drain une orientation particulière, inclinée vers le corps du patient. Il ne permet pas non plus le prélèvement d'éventuelles sécrétions qui apparaîtraient au niveau de l'orifice dans le corps du patient.

Il en est de même pour le connecteur rigide décrit dans le document WO 97/45148.

EP-A-82 596 décrit un dispositif de maintien d'un drain sans protubérance ni bourrelet de fixation.

L'invention a pour but de pallier ces inconvénients en proposant un dispositif de maintien d'au moins un drain ou analogue tel qu'une sonde, une lame ou un drain microtubulé, permettant de courber et orienter le drain à sa sortie de l'orifice dans le corps du patient, sans générer d'effort et donc de douleur pour le patient et sans entraîner de risque de déplacement du drain.

Ainsi, l'invention concerne un dispositif de maintien d'au moins un drain ou analogue passant par une cavité artificielle dans le corps d'un patient, caractérisé en ce qu'il comprend au moins une chambre de collection pour le recueil d'éventuelles sécrétions, une embase destinée à être fixée audit corps et entourant chaque chambre de collection, ainsi qu'au moins un orifice dans la chambre de collection pour le passage d'un drain ou analogue, l'axe dudit orifice n'étant pas perpendiculaire au plan de l'embase, pour pouvoir courber le drain et ainsi l'orienter par rapport au corps du patient.

De préférence, l'orifice pour le drain se prolonge par un support comportant un évidement pour le drain.

Dans un premier mode de réalisation, l'évidement du support est orienté de telle sorte que son axe correspond sensiblement à celui de l'orifice.

Dans un deuxième mode de réalisation, l'évidement du support est orienté de telle sorte que son axe est incliné par rapport à celui de l'orifice.

De préférence, le support pour le drain est constitué de deux parties articulées.

Le support pour le drain comporte avantageusement des moyens coopérant avec le drain pour le fixer en position par rapport au dispositif.

Dans une variante de réalisation, ces moyens de fixation sont constitués par une jupe, dans le prolongement du support, cette jupe convergeant vers l'axe de l'évidement du support et étant destinée à s'encliqueter dans une rainure transversale prévue sur ledit drain, notamment au moyen du bourrelet qui la termine de préférence.

Dans une autre variante de réalisation, ces moyens de fixation sont constitués par au moins une protubérance prévue sur la paroi de l'évidement du support et destinée à s'encliqueter dans une rainure longitudinale ou transversale prévue sur le drain.

L'embase du dispositif selon l'invention comporte avantageusement une couche adhésive sur sa face destinée à venir en contact avec le corps d'un patient.

Dans un mode de réalisation, cette couche adhésive peut se prolonger vers l'extérieur de l'embase.

Dans un autre mode de réalisation, cette couche adhésive se prolonge au-delà de la partie intérieure de l'embase pour fermer partiellement la chambre de collection.

De préférence, la couche adhésive est réalisée en une gomme adhésive capable d'absorber des sécrétions.

De préférence, une chambre de collection du dispositif selon l'invention comporte également un orifice pour une poche de recueil de sécrétions.

De façon préférée, une chambre de collection du dispositif selon l'invention comporte au moins une fenêtre, notamment pour le prélèvement d'échantillons.

Par ailleurs, le dispositif selon l'invention est de préférence orienté sur le corps du patient pour favoriser le drainage par gravité.

Lorsque le dispositif comporte un orifice pour une poche de recueil de secrétions, celles-ci sont alors facilement drainées vers la poche.

L'invention sera mieux comprise et d'autres buts, avantages et caractéristiques de celle-ci apparaîtront plus clairement à la lecture de la description qui suit d'exemples non limitatifs de réalisation de l'invention, cette description étant faite au regard des dessins annexés, sur lesquels :
- la figure 1 est une vue en perspective d'un premier exemple de dispositif de maintien selon l'invention, avec un drain ;
- la figure 2 est une vue en coupe transversale selon II-II de la figure 1 ;
- la figure 3 est une vue en perspective d'un deuxième exemple de réalisation du dispositif de maintien selon l'invention, avec un drain ;
- la figure 4 est une vue en perspective d'un troisième exemple de réalisation d'un dispositif de maintien qui ne fait pas partie de l'invention, à l'état non utilisé ;
- la figure 5 est une vue en perspective du dispositif de maintien illustré à la figure 4, utilisé avec un drain ;
- la figure 6 est une vue en perspective d'un quatrième exemple de réalisation d'un dispositif de maintien qui ne fait pas partie de l'invention ; et
- la figure 7 est une vue en perspective d'un cinquième exemple de réalisation du dispositif de maintien selon l'invention.

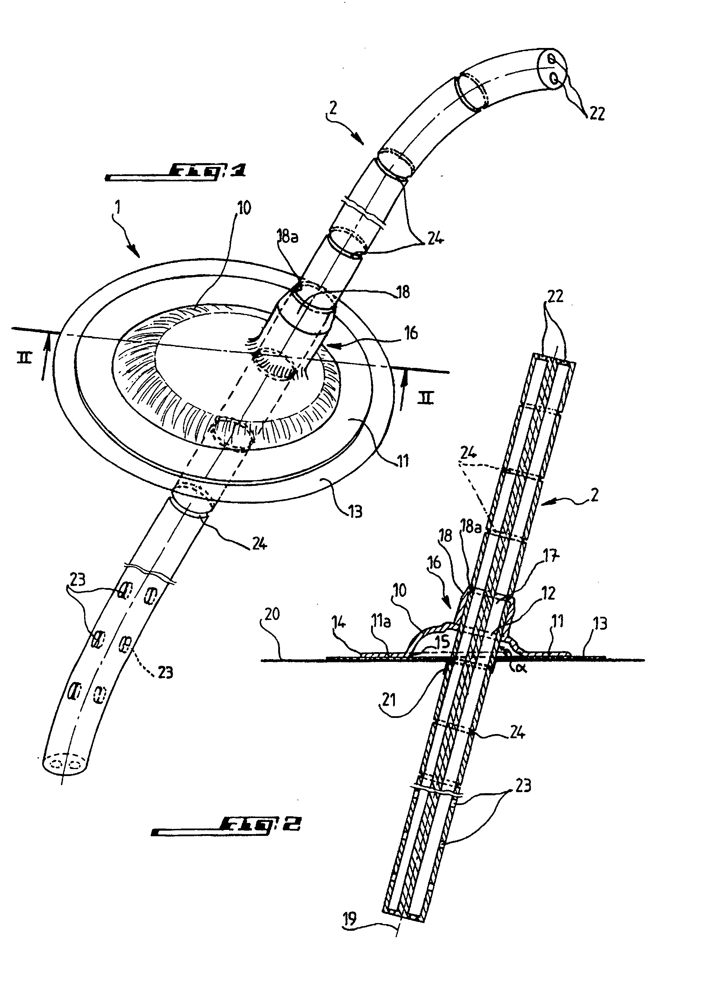

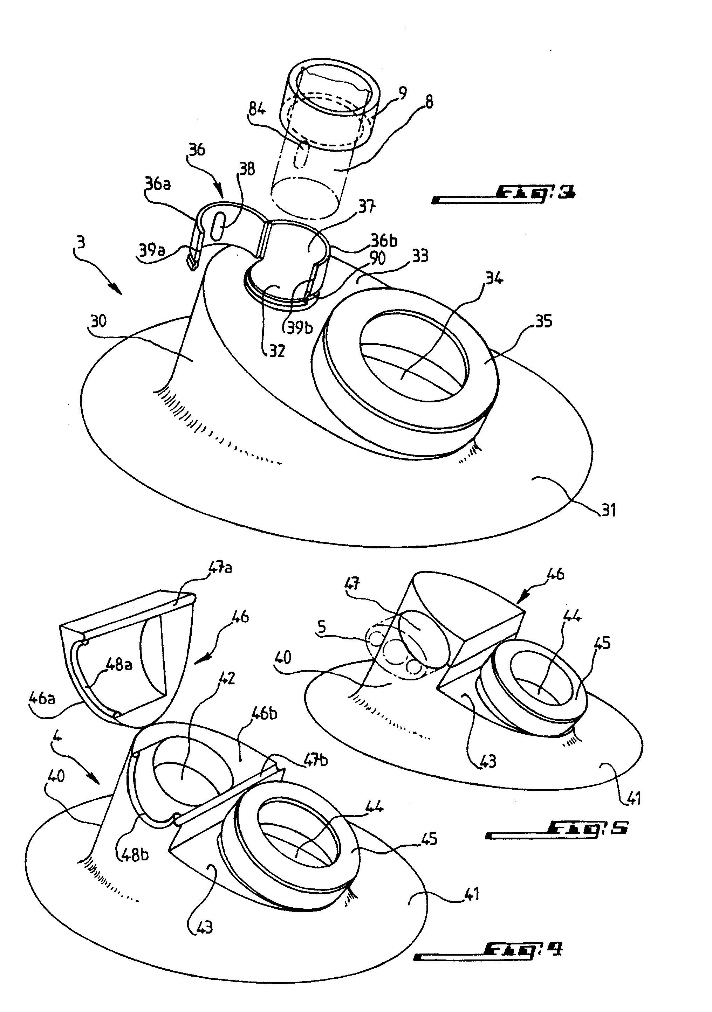

On se réfère tout d'abord aux figures 1 et 2 qui montrent un dispositif de maintien 1 selon l'invention comprenant une chambre de collection 10, une embase 11 entourant la chambre de collection 10 et un orifice 12 pour le passage d'un drain 2.

L'embase 11 est destinée à être fixée au corps d'un patient, notamment grâce à une couche adhésive 13 fixée à l'embase sur sa face 11a opposée à la chambre de collection 10.

Cette couche adhésive 13 peut être placée sur tout ou partie de la face 11a de l'embase 11. Elle peut également, comme illustré sur les figures 1 et 2, s'étendre au-delà de la périphérie extérieure 14 de l'embase 11 et également se prolonger au-delà de la partie intérieure 15 de l'embase, pour fermer partiellement la chambre de collection 10.

Comme le montre plus précisément la figure 2, dans ce cas, la couche adhésive ménage simplement un passage pour le drain 2.

De préférence, cette couche adhésive 13 est réalisée en une gomme adhésive capable d'absorber des sécrétions, par exemple une gomme naturelle du type pectine ou gélatine ou une gomme décrite dans le document EP-92999.

En pratique, cette couche adhésive 13 est protégée par un film approprié (non illustré) qui est retiré avant toute utilisation du dispositif 1.

Dans l'exemple de réalisation illustré aux figures 1 et 2, la chambre 10 présente une forme bombée ou en dôme. L'orifice 12 pour le drain 2 est réalisé dans la paroi de la chambre de collection 10, de telle sorte que l'axe 19 de l'orifice 12 ne soit pas perpendiculaire au plan de l'embase 11.

En pratique, l'orifice 12 n'est donc pas pratiqué dans la partie centrale du dôme de la chambre de collection 10, mais dans une position décalée.

L'utilisation du dispositif de maintien 1 selon l'invention est la suivante.

Un drain 2 est tout d'abord placé dans le corps d'un patient en passant par la cavité 21 pratiquée dans le corps.

Dans l'exemple illustré sur les figures, le drain 2 comporte deux canaux 22.

Dans sa partie inférieure, destinée à être placée dans le corps, le drain 2 comporte des perforations 23 pour la collecte des sécrétions.

Dans sa partie supérieure, destinée à être placée à l'extérieur du corps du patient, le drain 2 comporte des rainures transversales 24, continues, dont la fonction sera expliquée plus tard.

Une fois que le drain est en place dans le corps du patient, on fait coulisser le dispositif 1 le long de la partie supérieure du drain 2, jusqu'à ce que l'embase 11 vienne en appui sur le corps 20 du patient.

Le dispositif 1 est alors fixé au corps du patient au moyen de la couche adhésive 13.

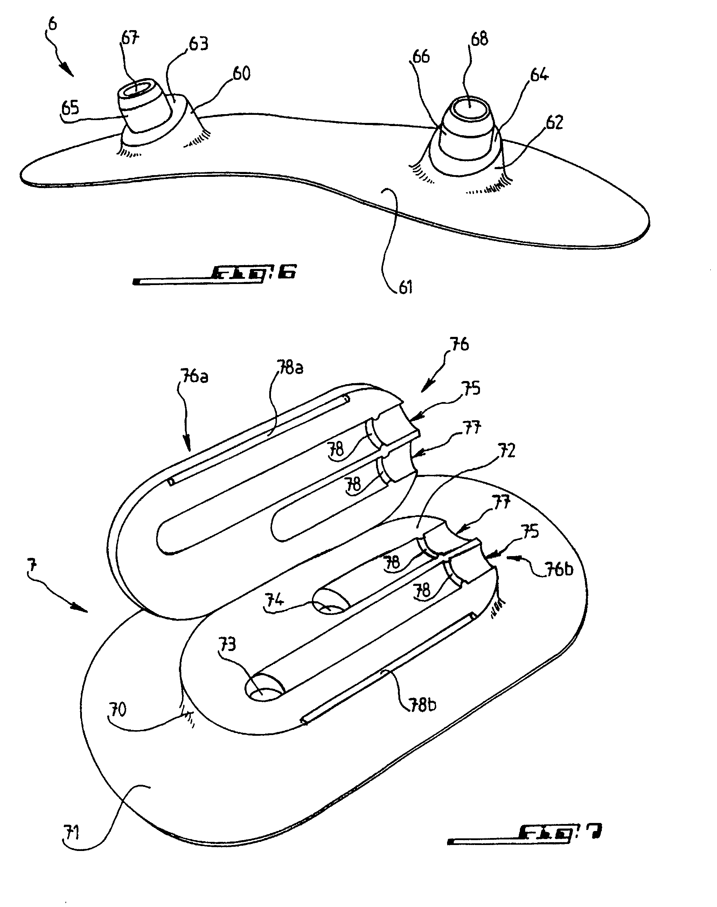

De préférence, le dispositif est fixé de telle sorte que la partie gauche de la figure 3 correspond à une partie supérieure du corps du patient et la partie droite à une partie inférieure.

Comme indiqué précédemment, l'axe de l'orifice 12 n'est pas perpendiculaire au plan de l'embase 11. De ce fait, en passant par l'orifice 12, le drain 2 n'est pas non plus perpendiculaire à l'embase 11.

De préférence, l'angle α inférieur à 90° que fait l'axe 19 de l'orifice 12 par rapport au plan de l'embase 11 est compris entre 5 et 85 degrés, et avantageusement entre 45 et 60°.

Grâce à cette inclinaison particulière de l'axe de l'orifice 12, le drain 2 peut être incliné et notamment rabattu vers le corps 20 du patient, sans que des efforts soient générés au niveau de la cavité 21.

Ainsi, la courbure imprimée au drain n'est pas douloureuse pour le patient et elle n'entraîne aucun déplacement du drain. Cette courbure est permise grâce à la présence de la chambre de collection.

Le dispositif de maintien illustré aux figures 1 et 2 comporte également un support 16 qui prolonge l'orifice 12 vers l'extérieur de la chambre de collection 10.

Ce support comporte un évidement 17 permettant le passage du drain.

Dans cet exemple de réalisation, le support 16 est disposé de telle sorte que l'axe de l'évidement 17 corresponde sensiblement à celui de l'orifice 12.

A son extrémité libre, le support 16 comporte une jupe 18 qui converge légèrement vers l'axe de l'évidement 17 et donc vers le drain, lorsque le dispositif 1 est placé sur celui-ci.

Cette jupe se termine, de préférence, par un bourrelet 18a qui est destiné à coopérer avec des rainures transversales 24 du drain pour mieux fixer le drain 2 par rapport au dispositif 1 et rendre l'assemblage étanche.

Lorsque l'on procède au retrait progressif du drain 2 du corps du patient, la jupe 18 s'encliquette sur des rainures différentes.

Au cours du drainage, des sécrétions peuvent s'accumuler autour de l'orifice 21. Il peut s'agir de liquides provenant de l'intérieur du corps du patient ou encore de saignements produits autour de l'ouverture 21.

Ces sécrétions sont ainsi recueillies à l'intérieur de la chambre de collection 10.

Par ailleurs, elles peuvent être à l'origine d'infections et il donc utile de pouvoir surveiller la présence de telles sécrétions et également de procéder à des prélèvements pour les analyser.

C'est pourquoi, il est avantageusement prévu dans la paroi de la chambre de collection 10, une fenêtre (non illustrée sur les dessins). Cette fenêtre peut notamment être fermée par une membrane ou un bouchon transparent.

Si le dispositif est fixé au corps comme indiqué précédemment, les secrétions s'accumulent dans la partie basse de la chambre de collection (à droite sur la figure 3) et on prévoit de préférence la fenêtre à ce niveau.

Le dispositif de maintien 1 illustré aux figures 1 et 2 peut être utilisé en chirurgie thoracique ou abdominale.

Le drain n'est pas nécessairement de section ronde, mais peut aussi être de section ovale.

D'autres exemples de réalisation du dispositif de maintien selon l'invention vont être décrits en référence aux figures 3 à 7. Les caractéristiques et avantages communs avec l'exemple décrit en référence aux figures 1 et 2 ne seront pas décrits de nouveau.

On se réfère maintenant à la figure 3 qui illustre un deuxième exemple de réalisation du dispositif de maintien selon l'invention.

Ce dispositif 3 comporte également une chambre de collection 30 et une embase 31 l'entourant.

La chambre de collection présente une forme générale en tronc de cylindre.

Elle comporte sur sa face supérieure 33 un orifice 32 pour un drain 8 ou analogue.

L'orifice 32 se prolonge par un support 36 comportant un évidement 37 pour le drain 8.

Le support 36 est constitué en deux parties 36a et 36b articulées. Il est représenté en position ouverte sur la figure 3.

Cette figure montre que l'axe de l'évidement 37 comme celui de l'orifice 32 n'est pas perpendiculaire à l'embase 31, grâce à l'inclinaison de la face supérieure 33 de la chambre de collection 30 par rapport au plan de l'embase 31.

Le support 36 comporte, sur sa face intérieure, au moins une protubérance 38. Lorsqu'un drain 8 passe à travers l'orifice 32 et l'évidement 37, cette protubérance 38 sur la paroi de l'évidement 37 vient s'encliqueter dans une rainure longitudinale 84 prévue sur le drain 8.

Cette protubérance 38 permet donc de mieux fixer le drain 8 en position par rapport au dispositif 3.

Les parties articulées peuvent être fixées, en position fermée, par un moyen d'encliquetage. Dans l'exemple illustré à la figure 3, ce moyen est constitué d'une partie semi-cylindrique 39a prévue sur le bord latéral libre de la partie 36a du support et d'une rainure 39b prévue sur le bord latéral libre de la partie 36b du support et destinée à recevoir la partie semi-cylindrique 39a.

Elles peuvent également être fixées en position fermée, au moyen de la bague 9. Celle-ci vient alors s'encliqueter dans la rainure 90 prévue à la base du support 36.

Par ailleurs, la protubérance 38 peut être omise. Dans ce cas, le support 36 se prolonge, comme le support 16 illustré aux figures 1 et 2, par une jupe légèrement convergente vers l'axe de l'évidement 37. Un bourrelet peut également être prévu sur le bord annulaire libre de cette jupe. La jupe est alors destinée à s'encliqueter dans une rainure transversale prévue sur le drain. Ce mode de réalisation n'est pas illustré sur la figure 3.

Dans l'exemple illustré à la figure 3, un autre orifice 34 est ménagé sur la paroi supérieure 33 de la chambre de collection 30. Cet orifice se prolonge par une jupe 35, destinée à la fixation d'une poche de recueil de sécrétions (non illustrée).

Le dispositif de l'invention est, de préférence, orienté sur le corps d'un patient de façon à ce que l'orifice 34 soit situé plus bas que l'orifice 32. Ainsi, les éventuelles sécrétions s'accumulent par gravité dans le bas de la chambre de collection 30, ce qui facilite le drainage et l'évacuation dans la poche de recueil de sécrétions.

Le dispositif de maintien 3 peut notamment être utilisé en chirurgie abdominale ou thoracique, pour des drains de taille intermédiaire, comprise entre 5 et 7 millimètres.

Le dispositif 3 pourrait également ne pas comporter d'orifice 34 pour la fixation d'une poche de recueil de sécrétions.

Le dispositif 3 apporte des avantages similaires à ceux des dispositifs 1 illustrés aux figures 1 et 2.

De plus, le drain ou analogue placé dans le dispositif 3 peut présenter une épaisseur inférieure à celle du drain 2 utilisé avec le dispositif 1. En effet, il suffit que le drain 8 comporte une section suffisamment épaisse pour ménager la rainure 84, l'épaisseur du drain pouvant être par ailleurs plus faible. Le drain 8 est donc moins rigide que le drain 2 illustré aux figures 1 et 2. Il peut être de section ronde ou ovale.

Les figures 4 et 5 illustrent un troisième exemple de réalisation d'un dispositif de maintien qui ne fait pas partie de l'invention.

Ce dispositif 4 comporte, comme le dispositif 3, une chambre de collection 40 présentant la forme générale d'un tronc de cylindre, avec une embase 41 entourant la chambre 40.

La chambre de collection 40 comporte donc une face supérieure 43 qui est inclinée par rapport au plan de l'embase 41.

Dans cette face supérieure 43, est ménagé un orifice 42 pour un drain ou analogue et un orifice 44 pour une poche de recueil de sécrétions.

Comme indiqué précédemment pour le dispositif 3, l'orifice 44 et la jupe 45 pour la fixation de la poche de sécrétions pourraient être omis.

L'orifice 42 pour un drain ou analogue se prolonge par un support 46 qui est constitué de deux éléments 46a et 46b articulés l'un par rapport à l'autre.

Le support 46 qui se présente sous la forme d'un boîtier, est en position ouverte sur la figure 4 et en position fermée sur la figure 5.

Le support 46 comporte un évidement 47 pour le passage d'un drain 5.

Comme le montrent les figures 4 et 5, le support 46 est disposé par rapport à l'orifice 42, de telle sorte que l'axe de l'évidement 47 ne coincide pas avec celui de l'orifice 42, mais est au contraire incliné par rapport à celui-ci.

Quand le dispositif 4 est mis en place sur un drain 5 ou analogue, le support 46 est tout d'abord en position ouverte. Le drain 5 passe alors à travers l'orifice 42, le drain 5 n'étant pas orthogonal au plan de l'embase, grâce à l'inclinaison de l'orifice 42.

Après fixation du dispositif 3 sur le corps d'un patient, le support 46 est fermé en faisant basculer la partie 46a sur la partie 46b.

Le support 46 est maintenu en position fermée, par exemple par encliquetage. La partie supérieure 46a du support peut comporter alors, sur son bord longitudinal libre, une partie semi-cylindrique 47a et la partie inférieure 46b du support, également sur son bord longitudinal libre, une rainure 47b destinée à recevoir la partie semi-cylindrique 47a.

Le support 46 permet ainsi de donner au drain 5 une orientation différente de celle que le drain présente lorsqu'il est dans l'orifice 42.

En pratique, le support 46 permet ainsi de placer le drain 5 dans une position sensiblement parallèle au corps du patient.

Des moyens contribuant au maintien du drain dans le boîtier 46 peuvent être prévus sur la paroi de l'évidement 47.

Dans l'exemple illustré sur la figure 4, ces moyens sont constitués par un anneau ou bourrelet constitué en deux parties 48a et 48b et formé sur la paroi de l'évidement 47.

Cet anneau est destiné à s'encliqueter dans une rainure transversale (non illustrée) prévue sur le drain 5 pour contribuer à l'étanchéité de l'assemblage.

Le dispositif de maintien 4 peut également être utilisé en chirurgie thoracique ou abdominale pour des drains présentant une taille intermédiaire comprise entre 5 et 7 millimètres. Le drain peut indifféremment présenter une forme ovale ou ronde.

La figure 6 illustre un quatrième exemple de dispositif de maintien qui ne fait pas partie de l'invention.

Ce dispositif 6 comporte deux chambres de collection 60 et 62 avec une embase 61 entourant ces deux chambres.

Contrairement au dispositif illustré aux figures 1 à 5, l'embase 61 n'est pas plane mais présente une forme légèrement concave pour épouser la forme du corps du patient et notamment la forme de la cage thoracique.

Chaque chambre 60, 62 présente la forme générale d'un cylindre tronqué, dont la face supérieure 63, 64 est inclinée par rapport à l'embase 61.

Dans chaque face supérieure 63, 64, est ménagé un orifice pour un drain ou analogue qui se prolonge par un support 65, 66. Chaque support 65, 66 comporte un évidement 67, 68 pour un drain. Ces supports 65 et 66 ne seront pas décrits plus en détail, référence étant faite à la description qui précède.

Ainsi, l'axe de l'évidement 67 n'est pas perpendiculaire au plan de l'embase, ou encore au plan passant par la zone de raccord entre la chambre 60 et l'embase 61. Il en est de même pour l'évidement 68.

Dans l'exemple illustré à la figure 6, les chambres de collection 60 et 62 sont orientées de façon différentes par rapport à l'embase 61.

Ce dispositif de maintien 6 est donc notamment utilisé en chirurgie thoracique et pour des drains dont la dimension va de 3 à 10 millimètres. Un tel dispositif permet de fixer plusieurs drains sur une même embase.

Il présente également l'avantage de maintenir la position relative des drains, de placer plusieurs drains dans un même espace avec un encombrement minimum et d'apporter un renforcement du maintien sur le corps du patient.

La figure 7 illustre un cinquième exemple de réalisation du dispositif de maintien selon l'invention.

Ce dispositif 7 comporte également une chambre de collection 70 et une embase 71 entourant la chambre 70 et destinée à être fixée au corps d'un patient.

La chambre de collection 70 présente une face supérieure 72 qui est sensiblement parallèle à l'embase 71.

Dans cette face supérieure 72, sont ménagés deux orifices 73, 74 dont les axes sont, de préférence, non perpendiculaires au plan de l'embase.

Ces orifices 73, 74 présentent avantageusement une forme ovale ce qui ménage un jeu pour le passage d'un drain ou analogue à travers eux et facilite le positionnement du dispositif 7 sur le corps d'un patient et la courbure du drain.

Ces orifices 73, 74 se prolongent par un support 76 qui est commun aux deux orifices.

Ce support constitue un boîtier dont la partie inférieure 76b est ménagée dans la paroi supérieure 72 de la chambre de collection, tandis que la partie supérieure 76a constitue un couvercle, articulé sur la chambre de collection pour se refermer sur celle-ci.

Ce support est maintenu en position fermée, par exemple par encliquetage. La partie supérieure 76a formant couvercle peut comporter dans ce cas une partie semi-cylindrique 78a sur son bord longitudinal libre et une rainure correspondante 78b sur la partie inférieure 76b qui est destinée à recevoir la partie semi-cylindrique 78a.

Le boîtier 76 définit deux évidements 75, 77 prolongeant chaque orifice 73, 74.

Dans l'exemple illustré à la figure 7, le support 76 est réalisé de façon à ce que des drains ou analogues passant dans les orifices 73, 74 puis dans les évidements 75, 77 soient rendus sensiblement parallèles à l'embase 71 et donc au corps du patient.

C'est pourquoi le dispositif 7 est conçu pour des parties du corps qui sont destinées à venir en appui notamment sur un lit, pour éviter toute traction. Il pourra donc être par exemple utilisé en chirurgie du dos.

Ce dispositif 7 est surtout destiné à des drains de petite taille, comprise entre 3 et 5 millimètres.

Ainsi, le dispositif 7 permet de plier des drains sans exercer de traction et en les protégeant grâce au boîtier 76.

Le boîtier 76 pourrait être conçu pour donner d'autres orientations aux drains.

Ce boîtier en lui-même fixe les drains où analogues en position par rapport au dispositif 7. Cette fixation peut être renforcée au moyen d'anneaux ou bourrelets 78 prévus dans les évidements 75 et 77 et qui coopèrent avec des rainures transversales prévues sur les drains (non illustrés), leur coopération rendant l'assemblage étanche.

L'invention n'est bien sûr pas limitée aux modes de réalisation qui viennent d'être décrits. En particulier, un dispositif selon l'invention pourrait comporter plus de deux chambres de collection sur une même embase ou encore plus de deux orifices pour des drains sur une même chambre de connexion. De surcroît, la position relative de l'axe de l'ouverture pour un drain ou analogue et de l'axe du support du drain prolongeant cet orifice peut être quelconque et choisie en fonction de l'application souhaitée.

De plus, une ouverture pour la fixation d'une poche de recueil de sécrétions peut être prévue sur tout dispositif de maintien selon l'invention.

La description qui précède a essentiellement été faite pour des drains. Cependant, le dispositif selon l'invention peut également être utilisé pour le maintien de sondes, notamment nasogastriques ou encore de lames, qui comportent une structure ondulée ou des drains microtubulés, lesquels permettent l'évacuation de fluides par capillarité.

Des bouchons sont également prévus dont la forme est adaptée à celle de l'ouverture pour le drain ou de l'évidement du support pour le drain. Ces bouchons servent à fermer l'ouverture ou l'évidement quand le drain est retiré du corps du patient. Lorsque le dispositif selon l'invention est utilisé en chirurgie thoracique, ces bouchons présentent une longueur suffisante pour venir en contact avec la cavité dans le corps du patient et ainsi éviter toute entrée d'air.

Enfin, les signes de référence insérés après les caractéristiques techniques figurant dans les revendications ont pour seul but de faciliter la compréhension de ces dernières et ne sauraient en limiter la portée.

## Revendications

1. Dispositif de maintien d'au moins un drain ou analogue passant par une cavité artificielle dans le corps d'un patient, comprenant au moins une chambre de collection (10; 30; 70) pour le recueil d'éventuelles sécrétions, comportant une paroi latérale et une paroi supérieure de fermeture de la chambre de collection, une embase (11, 31, 71) destinée à être fixée audit corps et entourant la chambre de collection en étant solidaire de la paroi latérale de cette chambre, le drain pouvant traverser la chambre de collection et être introduit dans le corps du patient en étant maintenu par la chambre de collection de façon inclinée relativement au corps du patient, la paroi supérieure étant inclinée par rapport au plan de l'embase (11, 31, 71) et comportant un orifice (12; 32; 73; 74) de passage et de maintien du drain à travers la chambre de collection (2; 5; 8), l'ensemble comprenant les parois latérale et supérieure et l'embase (11, 31, 71) étant réalisé en une seule pièce pour permettre la mise en place de cet ensemble sur le corps du patient par coulissement de l'ensemble le long de la partie supérieure du drain (2) préalablement introduit dans le corps (20) du patient jusqu'à ce que l'embase (11, 31, 71) vienne en appui sur le corps (20) du patient et, ensuite, de courber le drain et ainsi l'orienter par rapport au corps (20) du patient suivant l'angle d'inclinaison de la paroi supérieure de la chambre de collection, l'orifice (12; 32; 42; 73; 74) de passage du drain (2) se prolongeant par un support (16; 36; 76) solidaire de la paroi supérieure de la chambre de collection et comportant un évidement de passage du drain, **caractérisé en ce que** ledit support comporte des moyens de fixation pouvant coopérer avec le drain (2) pour le fixer en position par rapport à l'ensemble, les moyens de fixation comprenant un bourrelet (18a) ou au moins une protubérance (38; 78) solidaire du support et pouvant s'encliqueter dans l'une des rainures transversales (24; 84) ou longitudinales (84) prévues sur le drain (2).

2. Dispositif selon la revendication 1, **caractérisé en ce que** ledit évidement (17, 37, 68) dudit support est orienté de telle sorte que son axe correspond sensiblement à celui de l'orifice (12; 32).

3. Dispositif selon la revendication 1, **caractérisé en ce que** ledit évidement (75; 77) dudit support est orienté de telle sorte que son axe est incliné par rapport à celui dudit orifice (73; 74).

4. Dispositif selon l'une des revendications précédentes, **caractérisé en ce que** ledit support (36, 76) est constitué de deux parties articulées.

5. Dispositif selon l'une des revendications précédentes, **caractérisé en ce que** lesdits moyens de fixation sont constitués par une jupe (18), dans le prolongement du support (16), cette jupe convergeant vers l'axe de l'évidement (17) dudit support et pouvant s'encliqueter dans l'une des rainures transversales précitées (24) prévues sur ledit drain (2), au moyen du bourrelet précité (18a) qui la termine.

6. Dispositif selon l'une des revendications 1 à 4, **caractérisé en ce que** la protubérance (38; 78) des moyens de fixation est prévue sur la paroi de l'évidement (37; 75; 77) dudit support (36, 76) et peut s'encliqueter dans une rainure longitudinale (84) ou transversale prévue sur ledit drain.

7. Dispositif selon l'une des revendications précédentes, **caractérisé en ce que** l'embase (11) comporte, sur sa face (11a) opposée à la chambre de collection (10), une couche adhésive (13) destinée à venir en contact avec le corps d'un patient.

8. Dispositif selon la revendication 9, **caractérisé en ce que** ladite couche adhésive (13) s'étend au-delà de la périphérie extérieure (14) de l'embase (11).

9. Dispositif selon la revendication 7 ou 8, **caractérisé en ce que** ladite couche adhésive (13) se prolonge à l'intérieur (15) de l'embase pour fermer partiellement la chambre de collection (10) et ménager un passage du drain.

10. Dispositif selon l'une des revendications 7 à 9, **caractérisé en ce que** ladite couche adhésive est réalisée en une gomme adhésive capable d'absorber des sécrétions.

11. Dispositif selon l'une des revendications 1 à 10, **caractérisé en ce que** la paroi supérieure (33) de la chambre de collection (30) comporte également un autre orifice (34) prolongé par une jupe (35) pour la fixation d'une poche de recueil de sécrétions.

12. Dispositif selon l'une des revendications 1 à 11, **caractérisé en ce que** la chambre de collection comporte au moins une fenêtre, notamment pour le prélèvement d'échantillons.

13. Dispositif selon la revendication 12, **caractérisé en ce que** l'autre orifice (34) de la chambre de collection est situé à un niveau plus bas que l'orifice (32) de passage du drain pour faciliter le drainage, vers la poche de recueil de sécrétions, de sécrétions s'accumulant par gravité en bas de la chambre de collection.

## Patentansprüche

1. Vorrichtung zur Halterung für mindestens eine medizinische Drain oder dergleichen, die durch eine künstliche Kavität in den Körper eines Patienten eindringt, mit zumindest einem Sammelraum (10; 30; 70) zum Aufnehmen eventueller Sekrete, mit einer Seitenwand und einer oberen Wand zum Verschließen des Sammelraums, einer Sitzfläche (11, 31, 71), die dazu bestimmt ist, am genannten Körper befestigt zu werden und den Sammelraum umgibt, indem sie fest mit der Seitenwand dieses Raums verbunden ist, wobei die Drain den Sammelraum durchsetzen und in den Körper des Patienten eingeführt werden kann, indem sie bezüglich des Körpers des Patienten geneigt vom Sammelraum gehaltert wird, wobei die obere Wand bezüglich der Ebene der Sitzfläche (11, 31, 71) geneigt ist und eine Öffnung (12; 32; 73; 74) zum Durchführen und Haltern der Drain durch den Sammelraum (2; 5; 8) enthält, wobei die Einheit aus Seitenwand, oberer Wand und Sitzfläche (11, 31, 71) einstückig ausgeführt ist, um das Einsetzen dieser Einheit am Körper des Patienten durch Gleitbewegung der Einheit entlang des oberen Teils der zuvor in den Körper (20) des Patienten eingeführten Drain (2) soweit zu gestatten, dass die Sitzfläche (11, 31, 71) in Anlage an den Körper (20) des Patienten gelangt und um anschließend ein Umbiegen der Drain und damit deren Ausrichtung gegenüber dem Körper (20) des Patienten gemäß dem Neigungswinkel der oberen Wand des Sammelraums zu gestatten, wobei die Öffnung (12; 32; 42; 73; 74) zum Durchführen der Drain (2) sich über einen Träger (16; 36; 76) fortsetzt, der fest mit der oberen Wand des Sammelraums verbunden ist und eine Ausnehmung zum Durchführen der Drain enthält, **dadurch gekennzeichnet, dass** der genannte Träger Befestigungsmittel enthält, die mit der Drain (2) zusammenwirken können, um diese in ihrer Stellung bezüglich der Einheit zu fixieren, wobei die Befestigungsmittel einen Wulst (18a) bzw. zumindest einen Vorsprung (38; 78) aufweisen, der fest mit dem Träger verbunden ist und in eine der an der Drain (2) vorgesehenen Quernuten (24; 84) oder Längsnuten (84) einschnappen kann.

2. Vorrichtung nach Anspruch 1, **dadurch gekennzeichnet, dass** die genannte Ausnehmung (17, 37, 68) des genannten Trägers so ausgerichtet ist, dass deren Achse im wesentlichen derjenigen der Öffnung (12; 32) entspricht.

3. Vorrichtung nach Anspruch 1, **dadurch gekennzeichnet, dass** die genannte Ausnehmung (75; 77) des genannten Trägers so ausgerichtet ist, dass deren Achse zu derjenigen der genannten Öffnung (73; 74) geneigt verläuft.

4. Vorrichtung nach einem der vorangehenden Ansprüche, **dadurch gekennzeichnet, dass** der genannte Träger (36, 76) aus zwei angelenkten Teilen besteht.

5. Vorrichtung nach einem der vorangehenden Ansprüche, **dadurch gekennzeichnet, dass** die genannten Befestigungsmittel aus einem Mantel (18) in Verlängerung des Trägers (16) bestehen, wobei dieser Mantel zur Achse der Ausnehmung (17) des genannten Trägers konvergiert und mittels des vorgenannten Wulstes (18a), der den genannten Mantel abschließt, in eine der vorgenannten in der genannten Drain (2) vorgesehenen Quernuten (24) einschnappen kann.

6. Vorrichtung nach einem der Ansprüche 1 bis 4, **dadurch gekennzeichnet, dass** der Vorsprung (38; 78) der Befestigungsmittel an der Wand der Ausnehmung (37; 75; 77) des genannten Trägers (36, 76) vorgesehen ist und in eine Längsnut (84) oder eine Quernut einschnappen kann, die an der genannten Drain vorgesehen ist.

7. Vorrichtung nach einem der vorangehenden Ansprüche, **dadurch gekennzeichnet, dass** die Sitzfläche (11) an ihrer dem Sammelraum (10) entgegengesetzten Seite (11a) eine Haftschicht (13) aufweist, die dazu bestimmt ist, mit dem Körper eines Patienten in Kontakt zu treten.

8. Vorrichtung nach Anspruch 9, **dadurch gekennzeichnet, dass** die genannte Haftschicht (13) sich über den Außenumfang (14) der Sitzfläche (11) hinaus erstreckt.

9. Vorrichtung nach Anspruch 7 oder 8, **dadurch gekennzeichnet, dass** die genannte Haftschicht (13) sich im Inneren (15) der Sitzfläche fortsetzt, um den Sammelraum (10) teilweise zu verschließen und einen Durchgang für die Drain auszusparen.

10. Vorrichtung nach einem der Ansprüche 7 bis 9, **dadurch gekennzeichnet, dass** die genannte Haftschicht aus Haftgummi besteht, der Sekrete absorbieren kann.

11. Vorrichtung nach einem der Ansprüche 1 bis 10, **dadurch gekennzeichnet, dass** die obere Wand (33) des Sammelraums (30) auch eine weitere Öffnung (34) aufweist, die über einen Mantel (35) zum Befestigen einer Sekretsammeltasche fortgesetzt wird.

12. Vorrichtung nach einem der Ansprüche 1 bis 11, **dadurch gekennzeichnet, dass** der Sammelraum zumindest ein Fenster aufweist, insbesondere zur Probenentnahme.

13. Vorrichtung nach Anspruch 12, **dadurch gekennzeichnet, dass** die weitere Öffnung (34) des Sammelraums sich in einer niedrigeren Ebene als die Öffnung (32) zum Durchführen der Drain befindet, um die Drainage von Sekreten zur Sekretsammeltasche zu erleichtern, die sich aufgrund der Schwerkraft unterhalb des Sammelraums ansammeln.

## Claims

1. Device for maintenance of at least one tube or similar passing through an artificial cavity in the body of a patient, consisting of at least one collection chamber (10; 30; 70) for collection of any secretions, comprising a side wall and a top wall to close off the collection chamber and a base (11, 31, 71) intended to be fixed to said body and enclosing the collection chamber by being integral with the side wall of said chamber, the tube being able to pass through the collection chamber and be introduced into the body of the patient while being held by the collection chamber inclined relative to the body of the patient, the top wall being inclined in relation to the plane of the base (11, 31, 71) and containing an orifice (12; 32; 73; 74) for passage and maintenance of the tube through the collection chamber (2; 5; 8), the assembly consisting of the side and top walls and the base (11, 31, 71) made in one piece to allow its positioning on the body of the patient by sliding it along the top part of the tube (2) previously introduced into the body (20) of the patient until the base (11, 31, 71) is supported on the body (20) of the patient and then bending of the tube and its orientation in relation to the body (20) of the patient according to the angle of inclination of the top wall of the collection chamber, the orifice (12; 32; 42; 73; 74) for passage of the tube (2) being extended by a holder (16; 36; 76) integral with the top wall of the collection chamber and containing a recess for passage of the tube, **characterized in that** the said holder has fixing means capable of operating with the tube (2) to fix it in position in relation to the assembly, said fixing means consisting of a flange (18a) or at least one protuberance (38; 78) integral with the holder that can lock into any of the transverse (24; 84) or longitudinal (84) grooves provided on the tube (2)

2. Device according to claim 1, **characterized in that** said recess (17, 37, 68) of said holder is oriented so that its axis corresponds largely to that of the orifice (12; 32).

3. Device according to claim 1, **characterized in that** said recess (75; 77) of said holder is oriented so that its axis is inclined in relation to that of said orifice (73; 74).

4. Device according to any of the previous claims, **characterized in that** said holder (36, 76) consists of two hinged parts.

5. Device according to any of the previous claims, **characterized in that** said fixing means consist of a skirt (18) in the extension of the holder (16), said skirt converging on the axis of the recess (17) of said holder and being able to lock into any of the above-mentioned transverse grooves (24) provided on said tube (2), by means of the above-mentioned flange (18a) at which it ends.

6. Device according to any of claims 1 to 4, **characterized in that** the protuberance (38; 78) of the fixing means is provided on the wall of the recess (37; 75; 77) of said holder (36, 76) and can lock into a longitudinal (84) or transverse groove provided on said tube.

7. Device according to any of the previous claims, **characterized in that** the base (11) contains, on its face (11a) opposite to the collection chamber (10), an adhesive layer (13) intended to come into contact with the body of a patient.

8. Device according to claim 9, **characterized in that** said adhesive layer (13) extends beyond the outer periphery (14) of the base (11).

9. Device according to claim 7 or 8, **characterized in that** said adhesive layer (13) extends to the inside (15) of the base to partially seal the collection chamber (10) make room for passage of the tube.

10. Device according to any of claims 7 to 9, **characterized in that** said adhesive layer is formed of an adhesive rubber capable of absorbing secretions.

11. Device according to any of claims 1 to 10, **characterized in that** the top wall (33) of the collection chamber (30) also contains another orifice (34) extended by a skirt (35) for fixing of a pocket to collect secretions.

12. Device according to any of claims 1 to 11, **characterized in that** the collection chamber contains at least one window, mainly in order to take samples.

13. Device according to claim 12, **characterized in that** the other orifice (34) of the collection chamber is situated at a lower level than the orifice (12) for passage of the tube to facilitate drainage to the secretion collection pocket of secretions accumulating by gravity at the bottom of the collection chamber.
